# EUROPÄISCHE PATENTANMELDUNG

(11) **EP 1 698 610 A1**
(43) Veröffentlichungstag der Anmeldung: **06.09.2006**
(21) Anmeldenummer: 06002691.1
(22) Anmeldetag: 10.02.2006
(51) Int. Cl.: C07C 45/42, C07C 47/544, A01N 35/04

(54) **Verfahren zur Herstellung von wässrigen ortho-Phthalaldehyd-Lösungen**

(30) Priorität: 02.03.2005 AT 3542005
(71) Anmelder: DSM Fine Chemicals Austria Nfg GmbH & Co KG, 4021 Linz (AT)
(72) Erfinder: Giselbrecht, Karlheinz, 4061 Pasching (AT); Hillisch, Wolfgang, 4060 Leonding (AT)
(74) Vertreter: Lindinger, Ingrid

(57) **Zusammenfassung**

Verfahren zur Herstellung von wässrigen ortho-Phthalaldehydlösungen, bei welchem ein Acetal des o-Phthalaldehydes in Wasser eingebracht und anschließend durch Zugabe einer Säure ein pH-Wert < 7 eingestellt wird, worauf sodann bei einer Temperatur zwischen 10°C und 90°C das Acetal durch Abspalten des entsprechenden Alkohols in den o-Phthalaldehyd überführt wird, sowie Verwendung von OPA-Acetalen zur Herstellung dieser Lösungen.

## Beschreibung

Die Erfindung betrifft ein Verfahren zur Herstellung von wässrigen ortho-Phthalaldehyd-Lösungen aus ortho-Phtalaldehyd-Acetalen.

Wässrige Lösungen von ortho-Phthalaldehyd (OPA) finden Anwendung im Biozidbereich. Insbesondere werden diese Lösungen im Bereich der hochwirksamen Desinfektion von Endoskopen, sowie zur Desinfektion von medizinischem Equipment verwendet.

Bisher wurden wässrige Lösungen von OPA durch Lösen einer entsprechenden Menge an OPA in Wasser hergestellt.
OPA ist ein Feststoff und liegt in kristalliner Form vor und weist in dieser Form einige Nachteile auf, da es giftig ist und zu Hautreizungen führt, sodass das Handling mit OPA erschwert wird und erhebliche Sicherheitsmaßnahmen bei der Formulierung der wässrigen OPA-Lösungen erforderlich sind. Weiters neigt OPA zum Verblocken, wodurch erstens beim Chargieren Probleme auftreten und zweitens langwierige Löseprozesse erforderlich sind.
OPA selbst wird, wie beispielsweise in EP 0 839 789 beschrieben, durch Verseifen von flüssigen OPA-Acetalen, die als Depotverbindungen für OPA dienen, hergestellt. Die Verseifung erfolgt gemäß obiger Literatur durch Hydrolyse bei einem pH-Wert zwischen 0 und 7 mittels Mineralsäuren, wie HCl, H₂SO₄, H₃PO₄ oder organischen Säuren, wie Essigsäure, Ameisensäure, p-Toluolsulfon- oder Methansulfonsäure.

Die Verwendung von OPA, beziehungsweise von OPA-Acetalen zur Herstellung von wässrigen OPA-Glutaraldehydlösungen, die ebenfalls im Biozidbereich eingesetzt werden, ist beispielsweise aus EP 0 843 966 bekannt, wonach eine wässrige OPA-Glutaraldehydlösung durch Spaltung eines OPA-Acetals in wässriger Glutaraldehydlösung und Entfernen des abgespaltenen Alkohols erhalten wird.

Aufgabe der vorliegenden Erfindung war es, wässrige OPA-Lösungen herzustellen, wobei die Nachteile des Einsatzes von kristallinem OPA vermieden werden.

Gegenstand der vorliegenden Erfindung ist demnach die Herstellung von wässrigen ortho-Phthalaldehydlösungen, das dadurch gekennzeichnet ist, dass ein Acetal des o-Phthalaldehydes in Wasser eingebracht und anschließend durch Zugabe einer Säure ein pH-Wert < 7 eingestellt wird, worauf sodann bei einer Temperatur zwischen 10°C und 90°C das Acetal durch Abspalten des entsprechenden Alkohols in den o-Phthalaldehyd überführt wird.

Bei dem erfindungsgemäßen Verfahren wird ein OPA-Acetal in Wasser eingebracht bzw. emulgiert und durch Hydrolyse gelöst. Als OPA-Acetal kommen Dialkylacetale (offenkettige Acetale) oder Dialkoxyphthalane (cyclische Acetale von OPA) mit 1 bis 6 C-Atomen im Alkylteil in Frage. Bevorzugt sind Dialkoxyphtalane oder Dialkylacetale mit 1 bis 4 C-Atomen im Alkylteil, besonders bevorzugt mit 1 bis 2 C-Atomen im Alkylteil.

Erfindungsgemäß wird soviel Acetal in Wasser eingebracht und gelöst, dass nach dem Ansäuern eine 0,025 bis 9 Gew.%ige wässrige OPA-Lösung erhalten wird. Bevorzugt sind 0,05 bis 5 Gew.%ige Lösungen, besonders bevorzugt 0,5 bis 2 Gew.%ige Lösungen.
Dabei ist zu beachten, dass das Gleichgewicht in der wässrigen Lösung laut 1 H-NMR zu 25% auf OPA und zu 75% auf OPA-Halb-Acetalen liegt.
Um höher konzentrierte Lösungen zu erhalten, ist es möglich, der Lösung einen Lösungsvermittler, wie etwa Polyethylenglykol oder mit Wasser mischbare Lösungsmittel aus der Gruppe der Ketone, wie etwa N-Methylpyrrolidon, Aceton, u.s.w., der Aldehyde, wie etwa Glutaraldehyd, u.s.w. zuzusetzen, wodurch OPA-Konzentrationen bis zu 50Gew% erhalten werden können.

Um das Acetal in OPA zu überführen, wird der wässrigen Lösung eine Säure zugesetzt, sodass ein pH-Wert von unter 7 eingestellt wird.
Als Säure kommen dabei Mineralsäuren, wie etwa Schwefelsäure, HCl u.s.w., oder organische Säuren, wie etwa Ameisensäure, Essigsäure, Glyoxylsäure, u.s.w. in Frage. Bevorzugt wird Schwefelsäure, Glyoxylsäure oder Ameisensäure eingesetzt.

Der pH-Wert sollte unter 7 sein, bevorzugt ist ein pH-Wert bis 5, besonders bevorzugt bis 3.
Die Spaltung erfolgt bei einer Temperatur von 10 bis 90°C, bevorzugt bei 20 bis 50°C. Bevorzugt wird die Lösung dabei gerührt.
Wird das Acetal in Wasser gegeben, liegt zuerst eine 2-phasige Emulsion vor, die sich erst nach Einstellen des pH-Werts auf unter 7 in eine homogene Lösung umwandelt.
Von Vorteil bei dem erfindungsgemäßen Verfahren ist, dass im Vergleich zur Verwendung von OPA selbst, höher konzentrierte Lösungen (9% im Vergleich zu 5%) hergestellt werden können, da der sich durch das Ansäuern abspaltende Alkohol das Auflösen des gebildeten OPAs begünstigt.

Gegebenenfalls kann nach der Spaltung die erhaltene wässrige OPA-Lösung neutralisiert bzw. auf einen pH-Wert von 4 bis 10, bevorzugt bis 8, eingestellt werden, beispielsweise durch Zugabe von anorg. Basen wie NaHCO3, NaOH, KOH, u.s.w.
Weiters kann gewünschtenfalls der sich abspaltende Alkohol durch Abdestillieren aus der Lösung entfernt werden, wodurch sich jedoch die Löslichkeit wieder auf 5% erniedrigt.

Weiters können der erfindungsgemäß hergestellten Lösung neben den bereits erwähnten Lösungsvermittlern noch andere übliche Hilfsstoffe, wie etwa Stabilisatoren, Antioxidantien, Duftstoffe, Farbstoffe, u.s.w., zugesetzt werden.

Die erfindungsgemäß hergestellten wässrigen OPA-Lösungen eignen sich besonders zum Einsatz im Biozidbereich und zeichnen sich insbesondere dadurch aus, dass erstens der abschließende Produktionsschritt für OPA durch den direkten Einsatz des Acetals entfällt, wodurch Kosten erspart werden und zweitens wesentliche Handlingvorteile durch die Verwendung des OPA-Acetals gegeben sind.

Die erfindungsgemäße Herstellung der wässrigen OPA-Lösungen kann aber auch in automatisierten Desinfektionsgeräten erfolgen.

Ein weiterer Gegenstand der Erfindung ist die Verwendung von OPA-Acetalen als Edukt für die Herstellung von wässrigen OPA-Lösungen, die im Biozidbereich eingesetzt werden.

### Beispiel 1:

Es wurden OPA-Lösungen mit unterschiedlichen Konzentrationen aus OPA-Dimethylacetal-Wasser-Säure-Mischungen hergestellt.
Die Reaktionsbedingungen (verwendete Säure, pH-Wert, Temperatur, Gew.%) und die Zeit, bis eine klare, homogene Lösung erhalten wurde (vollendete Spaltung) sind aus nachfolgenden Tabellen ersichtlich.

**Tabelle 1) pH: 1,0 mit Schwefelsäure:**

| A | B | Zeit bei 25°C bis klare Lösung (min.): | Zeit bei 35°C bis klare Lösung (min.): | Zeit bei 45°C bis klare Lösung (min.): |
|---|---|---|---|---|
| 0,7 | 0,5 | 15 | 7,5 | 3,75 |
| 1,3 | 1 | 15 | 7,5 | 3,75 |
| 2,7 | 2 | 30 | 15 | 7,5 |
| 4,0 | 3 | 40 | 20 | 10 |
| 5,4 | 4 | 45 | 22,5 | 11,25 |
| 6,7 | 5 | 60 | 30 | 15 |
| 8,1 | 6 | 80 | 40 | 20 |
| 9,4 | 7 | 90 | 45 | 22,5 |
| 10,7 | 8 | 150 | 75 | 37,5 |
| 13,4 | 9 | 330 | 165 | 82,5 |

| | | | | |
|---|---|---|---|---|
| **A: eingesetzte OPA-Acetal-Konzentration in Gew.%** | | | | |
| **B: OPA-Konzemtration in Lösung in Gew.%** | | | | |

**Tabelle 2) pH: 2,0 mit Schwefelsäure:**

| A | B | Zeit bei 25°C bis klare Lösung (min.): |
|---|---|---|
| 1,3 | 1 | 15 |
| 4,0 | 3 | 120 |
| 9,4 | 7 | 400 |

**Tabelle 3) pH: 2,0 mit Glyoxylsäure:**

| A | B | Zeit bei 25°C bis klare Lösung (min.): |
|---|---|---|
| 0,7 | 0,5 | 30 |
| 1,3 | 1 | 60 |
| 2,7 | 2 | 90 |
| 4,0 | 3 | 120 |
| 6,7 | 5 | 480 |

**Tabelle 4) pH: 2,0 mit Essigsäure:**

| A | B | Zeit bei 25°C bis klare Lösung (min.): |
|---|---|---|
| 0,7 | 0,5 | 15 |
| 1,3 | 1 | 20 |
| 2,7 | 2 | 60 |
| 4,0 | 3 | 120 |
| 5,4 | 4 | 240 |

**Tabelle 5) pH: 2,0 mit Ameisensäure:**

| A | B | Zeit bei 25°C bis klare Lösung (min.): |
|---|---|---|
| 1,3 | 1 | 20 |
| 2,7 | 2 | 90 |
| 4,0 | 3 | 150 |
| 5,4 | 4 | 300 |

## Patentansprüche

1. Verfahren zur Herstellung von wässrigen ortho-Phthalaldehydlösungen, das **dadurch gekennzeichnet ist, dass** ein Acetal des o-Phthalaldehydes in Wasser eingebracht und anschließend durch Zugabe einer Säure ein pH-Wert < 7 eingestellt wird, worauf sodann bei einer Temperatur zwischen 10°C und 90°C das Acetal durch Abspalten des entsprechenden Alkohols in den o-Phthalaldehyd überführt wird.

2. Verfahren nach Anspruch 1, **dadurch gekennzeichnet, dass** als Acetal des o-Phthalaldehydes ein Dialkylacetal oder ein Dialkoxyphthalan mit 1 bis 6 C-Atomen im Alkylteil eingesetzt wird.

3. Verfahren nach Anspruch 1, **dadurch gekennzeichnet, dass** als Säure eine Mineralsäure aus der Gruppe Schwefelsäure oder HCl oder eine organische Säure aus der Gruppe Ameisensäure, Essigsäure oder Glyoxylsäure eingesetzt wird.

4. Verfahren nach Anspruch 1, **dadurch gekennzeichnet, dass** der pH-Wert auf einen Wert bis 3 eingestellt wird.

5. Verfahren nach Anspruch 1, **dadurch gekennzeichnet, dass** eine 0,025 bis 9 Gew.%ige o-Phthalaldehydlösung hergestellt wird.

6. Verfahren nach einem der Ansprüche 1 bis 5, **dadurch gekennzeichnet, dass** der Lösung gegebenenfalls ein Lösungsvermittler aus der Gruppe Polyethylenglykol, N-Methylpyrrolidon, Aceton oder Glutaraldehyd zugesetzt wird.

7. Verfahren nach einem der Ansprüche 1 bis 6, **dadurch gekennzeichnet, dass** der Lösung gegebenenfalls weitere Hilfsstoffe zugesetzt werden.

8. Verfahren nach einem der Ansprüche 1 bis 7, **dadurch gekennzeichnet, dass** die Lösung gegebenenfalls nach der Spaltung durch Zugabe einer Base auf einen pH-Wert von 4 bis 10 gestellt wird.

9. Verfahren nach einem der Ansprüche 1 bis 8, **dadurch gekennzeichnet, dass** die Herstellung in einem automatisierten Desinfektionsgerät erfolgt.

10. Verwendung von OPA-Acetalen als Edukt für die Herstellung von wässrigen OPA-Lösungen, die im Biozidbereich eingesetzt werden.
